# EUROPEAN PATENT SPECIFICATION

(11) **EP 1 564 302 B1**
(45) Date of publication and mention of the grant of the patent: **14.09.2011**
(21) Application number: 04003301.1
(22) Date of filing: 13.02.2004
(51) Int. Cl.: C12Q 1/68, G01N 33/574

(54) **Genes for diagnosing colorectal cancer**
Gene zur Diagnose von kolorektal Krebs
Genes pour le diagnostic du cancer du colon

(43) Date of publication of application: 17.08.2005
(73) Proprietor: Kaohsiung Medical University, Kaohsiung City (TW)
(72) Inventor: Lin, Shiu-Ru, Kaohsiung City, Taiwan 807 (TW); Wang, Jaw-Yuan, Kaohsiung City, Taiwan 813 (TW)
(74) Representative: Beck, Michael Rudolf

(56) References cited:
- DATABASE BIOSIS [Online] BIOSCIENCES INFORMATION SERVICE, PHILADELPHIA, PA, US; 2002, LIN SHIU-RU ET AL: "Identification of genes overexpressed in tumorigenesis of human colorectal cancers by combining suppression subtractive hybridization and microarray analysis" XP009034492 Database accession no. PREV200200596316 & INTERNATIONAL JOURNAL OF MOLECULAR MEDICINE, vol. 10, no. Supplement 1, 2002, page S46, 7TH WORLD CONGRESS ON ADVANCES IN ONCOLOGY AND THE 5TH INTERNATIONAL SYMPOSIUM ON MOLECULAR MEDICINE; HERSONISSOS, CRETE, GREECE; OCTOBER 10-12, 2002 ISSN: 1107-3756
- SWEARINGEN M L ET AL: "Detection of differentially expressed HES-6 gene in metastatic colon carcinoma by combination of suppression subtractive hybridization and cDNA library array" CANCER LETTERS 2003 IRELAND, vol. 198, no. 2, 2003, pages 229-239, XP002290172 ISSN: 0304-3835
- SAITO A ET AL: "Detection of genes expressed in primary colon cancers by in situ hybridisation: Overexpression of RACK 1" JOURNAL OF CLINICAL PATHOLOGY - MOLECULAR PATHOLOGY 2002 UNITED KINGDOM, vol. 55, no. 1, 2002, pages 34-39, XP002290173 ISSN: 1366-8714
- NAKAGAWA MASAHIRO ET AL: "Polymorphic expression of decay-accelerating factor in human colorectal cancer" JOURNAL OF GASTROENTEROLOGY AND HEPATOLOGY, vol. 16, no. 2, February 2001 (2001-02), pages 184-189, XP002290174 ISSN: 0815-9319
- DATABASE BIOSIS [Online] BIOSCIENCES INFORMATION SERVICE, PHILADELPHIA, PA, US; 15 March 1999 (1999-03-15), YANG GEORGE P ET AL: "Combining SSH and cDNA microarrays for rapid identification of differentially expressed genes" XP002290175 Database accession no. PREV199900212235 & NUCLEIC ACIDS RESEARCH, vol. 27, no. 6, 15 March 1999 (1999-03-15), pages 1517-1523, ISSN: 0305-1048
- DATABASE EMBL ALL [Online] 9 April 2002 (2002-04-09), XP002290262 retrieved from EBI Database accession no. BC027178

## Description

This invention relates to a set of oligonucleotides for diagnosing colorectal cancer particularly suited for a method of clinical diagnosis for colorectal cancer which enables the effects of early diagnosis, specificity, highly sensitivity and safety.

Colorectal cancer is one of the most common malignant tumors in the world. It is the second most frequent cause of malignant tumor related mortality in developed countries. In developed countries, mortality rate caused by colorectal cancer seems have a descending tendency progressively in previous 20 years, the main causes for early diagnosis is provided and the improvement of methods of therapy and medicines. But in Taiwan because of changes in diet habit to occidental habit and the rapid changes in environment, the rate of suffering from colorectal cancer is rising constantly, furthermore, also showing an age-descending tendency.

Amongst the top ten types of cancer of Taiwanese of 2002, colorectal cancer (CRC) is the third leading cause of cancer-related death for male and female, which is announced by The Department of Health (DOH), highest level of the executive branch, Taiwan. About 6681 new cases of colorectal cancer diagnosed per year such as statistical data by DOH of 1999, for 3649 patients dead in the colorectal cancer per year such as statistical data by DOH of 2002 in Taiwan. The average age of colorectal cancer patients is lower than in other countries. In other words, twenty-year-old or thirty-year-old people suffer from colorectal cancer in Taiwan. Therefore, we cannot ignore the possibility of the colorectal cancer in young persons.

Although methods of diagnosis and surgical operation treatment are improved for colorectal cancer patients, when a comparison is made between early diagnosis with later period diagnosis by surgical operation respectively, the treatment is able to probably overcome the colorectal cancer in early diagnosis, but is not able to absolutely overcome the colorectal cancer in the later period diagnosis. The far metastasis are the main problem of treatment of colorectal cancer, therefore, a method with high sensitivity, high specificity and easy diagnosis which is able to detect early and potentially curable CRCis believed to be a novel target for CRC diagnosis and therapy.

The present invention aims at providing a set of oligonucleotides for early diagnosing possibility of recurrence and metastasis for colorectal patients. Simultaneously, tracing 100 colorectal cancer cases, found that 92% genes variation in colorectal tissue. In the process of tracing for 100 colorectal cancer cases simultaneously, mutation of genes is found in 92% colorectal cancer tissues. In the tracing process, although CEA of 16 patients still in normal value range, that detect early tumor cells in blood by using genes variation testing.

Swearingen, M. L. et. al, Cancer Letters, 2003, Vol. 198, p. 229-239 disclose HES-6 as a marker for metastases of colon carcinoma (cell line HT29). The metastases were dissected from lung tissue of mice rather than from human beings. In the corresponding study a colorectal cancer cell line from ATCC was injected into the mouse body to find a difference between a metastatic tissue and a non-metastatic tissue.

Lin, Shiu-Ru et al., International Journal of Molecular Medicine, 2002, Vol. 10, Supp. 1, p. S46 confirm the 15 most related genes to carcinogenesis of colorectal cancer without examining their expression in clinical patients' tissue to confirm their high relation to the process of carcinogenesis of colorectal cancer.

Sequence BC027178 could be identified in database EMBL. However, the database was silent on any specific function.

Marker genes and oligoncleotides specific thereto for the diagnosis of colorectal cancer are known from WO 00/55351, Nakagawa, M. et al., Gastroenterology and Hepatology, 2001, vol. 16, p. 184-189, and Hedge, P. et al., Cancer Research, 2001, vol. 61, p. 7792-7797.

WO 00/55351to Rosen, C. A. et al., "Human Colon Cancer Associated Gene Sequences And Polypeptides", discloses colon cancer related polynucleotides and the polypeptides encoded by the polynucleotides herein collectively known as "colon cancer antigens", screening methods for identifying agonists and antagonists of colon cancer antigens of the invention. The present application describes a SSH and cDNA microarray technology for the identification of candidate marker genes which are overexpressed continuously from colorectal proliferous polypus to colorectal oncogene, overexpressed genes are selected from up regulation genes which related intently in colorectal cancer oncogene, and down regulation genes which related in colorectal cancer oncogene. The total 71 genes are used to diagnosing early colorectal cancer.

Therefore, the main purpose according to the present application is to provide marker genes for clinical diagnosis of colorectal cancer for early diagnosis, specificity, highly sensitivity and safety.

The invention provides a set of oligonucleotides for diagnosing colorectal cancer according to claim 1. Advantageous embodiments are laid down in further claims.

For the purpose stated above, the identification of genes comprises the steps of: (1) deriving epithelium cells from normal intestines, polypus of intestines and colorectal cancer tissue; (2) collecting genes with highly differential gene expression by Suppression Subtractive Hybridization (SSH), and building a library; (3) deriving colonies with relatively high signal intensities from cancer tissue; (4) collecting more clinically cancer tissues by Northern Hybridization, real-time Polymerase Chain Reaction (PCR) combined with analysis of bioinformation to affirm variation between differential gene expression; and (5) selecting the most suitable genes from said library. Moreover, the reagent uses the gene sequence as method of clinical diagnosis for colorectal cancer to the early diagnosis.

The present invention will be better understood from the following detailed description of preferred embodiments of the invention, taken in conjunction with the accompanying drawings, in which:
- Table 1: is a table showing the result of clinical examination of colorectal cancer biochip;
- FIG. 1: is a view showing the procedure of deriving genes according to the method described in the present application ;
- FIG. 2a and FIG. 2b: are views showing the primary screening according to the method described in the present application ;
- FIG. 3a and FIG.3b: are views showing affirmation to genes using Northern Blotting method according to the method of present application ;
- FIG. 4a and FIG. 4b: are views showing quantity expression of cancer tissue according to the method of present application; and
- FIG.5: is a diagram showing second preferred embodiments according to the present disclosure.

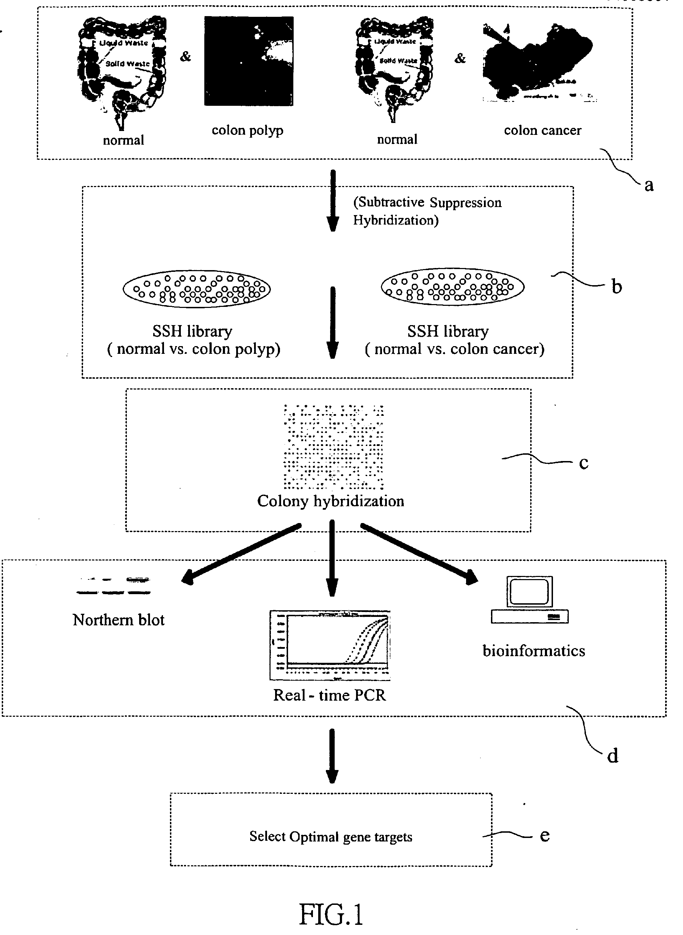

The following descriptions of the preferred embodiments are provided to understand the methods and the procedures of the present disclosure. Please refer to FIG.1, showing the procedure of searching genes according to the present disclosure. Said procedure comprise the steps of: (1) deriving epithelium cells from normal intestines, polypus of intestines and colorectal cancer tissue; (2) collecting genes with highly differential gene expression by Suppression Subtractive Hybridization (SSH), and building library ; (3) deriving colonies with relatively high signal intensities from cancer tissue; (4) collecting more clinically cancer tissues by Northern Hybridization, real-time Polymerase Chain Reaction (PCR) combined with analysis of bioinformation to affirm variation between differential gene expression; and (5) selecting the most suitable genes from said library. Moreover, by using the gene sequence as a reagent, this enables clinical diagnosis for colorectal cancer to the effects of early diagnosis, specificity, highly sensitivity and safety.

The genes for diagnosing colorectal cancer, the specific oligonucleotides sequence are selected from the group consisting of:

| SEQ ID NO | HS ID | Acc No | Discription | Definition | Oligo sequence |
|---|---|---|---|---|---|
| 1 | Hs.107213 | BC027178 | FNBP3 Formin binding protein 3 | Homo sapiens, formin binding protein 3, clone MGC:16979 IMAGE:4343048, mRNA, complete cds | CATCATAGGAAACGT TCCCGCTCTCGATC GGGGTCAGATTCAG ATGATGATG |
| 2 | Hs.123107 | NM_002257 | KLK1 Kallikrein 1, renal/pancreas/sal ivary | Homo sapiens kallikrein 1, renal/pancreas/salivary (KLK1), mRNA. | GCCTTCTGTCGCCG TCAGAGTGCTGTCTT ATGTGAAGTGGATC GAGGACA |
| 3 | Hs.1369 | NM_000574 for | DAF Decay accelerating factor complement (CD55, Cromer blood group system) | Homo sapiens decay accelerating factor for complement (CD55. Cromer blood group system) (DAF), mRNA | GGGCAGTCAATGGT CAGATATTGAAGAGT TCTGCAATCGTAGCT GCGAGGTG |
| 4 | Hs.151254 | NM_005046 | KLK7 Kallikrein 7 (chymotryptic, stratum corneum) | Homo sapiens kallikrein 7 (chymotryptic, stratum corneum) (KLK7), transcript variant 1, mRNA. | TGGAACCACCTGTA CTGTCTCCGGCTGG GGCACTACCACGA |
| 5 | Hs.1526 | NM_001681 | ATP2A2 ATPase, Ca++ transporting, cardiac muscle, slow twitch 2 | Homo sapiens ATPase, Ca++ transporting, cardiac muscle, slow twitch 2 (ATP2A2), mRNA | CATCGGCATCTTCG GGCAGGATGAGGAC GTGACGTCAAAAGC TTTCACAG |
| 6 | Hs.184270 | NM_006135 | CAPZA1 Capping protein (actin filament) muscle Z-line, alpha 1 | Homo sapiens capping protein (actin filament) muscle Z-line, alpha 1 (CAPZA1), mRNA. | TGACCACTTACGGA AAGAAGCAAGTGAC CCCCAGCCAGAAGA AGCAGATG |
| 7 | Hs 2043 | NM_001151 | SLC25A4Solute carrier family 25 (mitochondrial carrier; adenine nucleotide translocator), member 4 | Homo sapiens solute carrier family 25 (mitochondrial carrier; adenine nucleotide translocator), member 4 (SLC25A4), nuclear gene encoding mitochondrial protein, mRNA. | AGATCTTCAAGTCTG ATGGCCTGAGGGGG CTCTACCAGGGTTTC AACGTC |
| 8 | Hs.267871 | NM_005177 | ATP6VOA1 ATPase, H+ transporting, lysosomal V0 subunit a isoform 1 | Homo sapiens ATPase, H+ transporting, lysosomal V0 subunit a isoform 1 (ATP6VOA1), mRNA. | GGACAGAAAGGAAT TCAGTGTTTCCTGGT AGTGGTTGCACTACT GTGTGTACCTTGG |
| 9 | Hs.4935 | D79998 | KIAA0176 protein | Human mRNA for KIAA0176 gene, partial cds | GGAAAGGATACGGG ACAATGAGAACAGA ACTTCACAAGGCCC CGTGAAGC |
| 10 | Hs.5509 | NM_006495 | EVI2B Ecotropic viral integration site 2B | Homo sapiens ecotropic viral integration site 2B (EVI2B), mRNA. | GCCCCTGCCACCAG TAGATTTTATGAAAA ACCAAGAAGATTCCA ACCTTGAGATCCAGT GTC |
| 11 | Hs.5662 | NM_006098 | GNB2L1 Guanine nucleotide binding protein (G protein), beta polypeptide 2-like 1 | Homo sapiens guanine nucleotide binding protein (G protein), beta polypeptide 2-like 1 (GNB2L1), mRNA. | ATGACTGAGCAGAT GACCCTTCGTGGCA CCCTCAAGGCCAC AAC |
| 12 | Hs.75990 | NM_005143 | HP Haptoglobin | Homo sapiens haptoglobin (HP), mRNA. | AGGCTGTTGGAGAT AAACTTCCTGAATGT GAAGCAGATGACGG CTGCCCG |
| 13 | Hs.83384 | NM_006272 | S100B S100 calcium binding protein, beta (neural) | Homo sapiens S100 calcium binding protein, beta (neural) (S100B), mRNA | CCGAACTCAAGGAG CTCATCAACAATGAG CTTTCCCATTTCTTA GAGGAAATCAAAGA GCAGGAG |
| 14 | Hs.10029 | NM_001814 | CTSC Cathepsin C | Homo sapiens cathepsin C (CTSC), mRNA | CACCGGAAAGAAGG TGGGAACTGCCTCT GAGAATGTGTATGTC AACACAGC |
| 15 | Hs.103982 | NM_005409 | SCYB11 Small inducible cytokine subfamily B (Cys-X-Cys), member 11 | Homo sapiens small inducible cytokine subfamily B (Cys-X-Cys), member 11 (SCYB11), mRNA. | [?]GGGCATGGCTATAG CCTTGGCTGTGATAT TGTGTGCTACAGTTG TTCAAGGC |
| 16 | Hs.12314 | AL049397 | Homo sapiens mRNA; cDNA DKFZp586C1019 (from clone DKFZp586C1019) | Homo sapiens mRNA; cDNA DKFZp586C1019 (from clone DKFZp586C1019) | CAACACCACAGACA GCTGCAGGACTCGA TATCCATGGCTTCTT TCCATCAC |
| 17 | Hs.150557 | NM_001206 | BTEB1 Basic transcription element binding protein 1 | Homo sapiens basic transcription element binding protein 1 (BTEB1), mRNA. | TTCCACCCCAGCAT GATCAAGCGATCGA AAAAGGCGCTGGCC AACGCTTT |
| 18 | Hs.169266 | NM_000909 | NPY1 R Neuropeptide Y receptor Y1 | Homo sapiens neuropeptide Y receptor Y1 (NPY1 R), mRNA. | CCGGTCTCGGGATG ATGATTATGAAACAA TAGCCATGTCCACG ATGCACACAG |
| 19 | Hs.1827 | NM_002507 | NGFR Nerve growth factor receptor (TNFR superfamily, member 16) | Homo sapiens nerve growth factor receptor (TNFR superfamily, member 16) (NGFR), mRNA. | CAAGCGGGAGGAGG TGGAGAAGCTTCTC AACGGCTCTGCG |
| 20 | Hs.1869 | NM_002633 | PGM1 Phosphoglucomut ase 1 | Homo sapiens phosphoglucomutase 1 (PGM1), mRNA. | GCCAACGGGATCGG TCGCTTGGTTATCG GACAGAATGGAATC CTCTCCA |
| 21 | Hs.194148 | NM_005433 | YES1 V-yes-1 Yamaguchi sarcoma viral oncogene homolog 1 | Homo sapiens v-yes-1 Yamaguchi sarcoma viral oncogene homolog 1 (YES1), mRNA | CAAGTGTGAGCCAT TATGGAGCAGAACC CACTACAGTGTCAC CATGTCCG |
| 22 | Hs.2352 | X74210 | ADCY2 Adenylate cyclase 2 (brain) | H.sapiens mRNA for adenylyl cyclase | TCGTCTGCTTTGCTG GACAGCTTCTGCAAT GCAGCAAAAAAGCC TCTCCC |
| 23 | Hs.246885 | NM_017958 | FLJ20783 hypothetical protein FLJ20783 | Homo sapiens hypothetical protein FLJ20783 (FLJ20783), mRNA. | CCAAGATTCTAGGA CAAACACAGCGTAT GTGGGCTCTGCAGT CATGACCG |
| 24 | Hs.29665 | NM_014944 | CLSTN1 Calsyntenin 1 | Homo sapiens calsyntenin 1 (CLSTN1), mRNA. | CACGAGCCCTTCTC TGTGACTGAGGATTA CCCGCTCCATCCAT CCAAGAT |
| 25 | Hs.3235 | NM_002272 | KRT4 Keratin 4 | Homo sapiens keratin 4 (KRT4), mRNA | TTCAGCTGTGGCTC GGCCATTGTAGGCG GTGGCAAGAGAGGT |
| 26 | Hs.55209 | AF327354 | Homo sapiens DMR protein mRNA, complete cds | Homo sapiens DMR protein mRNA, complete cds | TAAAGTGGGCTCATT GTCATCCCCAAGCC AGGCCAGTTCTCCA GGTGGAA |
| 27 | Hs.585 | NM_000384 | APOB Apolipoprotein B (including Ag(x) antigen) | Homo sapiens apolipoprotein B (including Ag(x) antigen) (APOB), mRNA | GCCCAAGGCCACAG GGGTCCTTTATGATT ATGTCAACAAGTACC ACTGGG |
| 28 | Hs.62187 | AF022913 ol | PIGK Phosphatidylinosit glycan, class K | Homo sapiens GPI transamidase mRNA, complete cds | TCTTGTCCTTCGGCA GCGTGGCCGCTAGT CATATCGAGGATCAA GCAGAA |
| 29 | Hs.63290 | NM_012260 | HPCL2 2-hydroxyphytano yl-CoA lyase | Homo sapiens 2-hydroxyphytanoyl-Co A lyase (HPCL2), mRNA | CATGAACTGCTGGC CCTTGCTTGTGATTG GTGGTTCCTCTGAAA GAAACCAAG |
| 30 | Hs.699 | NM_000942 | PPIB Peptidylprolyl isomerase B (cyclophilin B) | Homo sapiens peptidylprolyl isomerase B (cyclophilin B) (PPIB), mRNA | AGCCGGGATAAACC CCTGAAGGATGTGA TCATCGCAGACTGC GGCAAGAT |
| 31 | Hs.74111 | NM_007367 d | RALY RNA binding protein (autoantigenic, hnRNP-associate with lethal yellow) | Homo sapiens RNA binding protein (autoantigenic, hnRNP-associated with lethal yellow) (RALY) transcript variant 2, mRNA | AGCGAGGAAGAGCT GGAACACAGCCAGG ACACAGACGCGGAT GAT |
| 32 | Hs.75103 | NM_003406 e | YWHAZ Tyrosine 3-monooxygenas e/tryptophan 5-monooxygenas activation protein, zeta polypeptide | Homo sapiens tyrosine 3-monooxygenase/tryp tophan 5-monooxygenase activation protein, zeta polypeptide (YWHAZ), mRNA | CGGAAGGTGCTGAG AAAAAACAGCAGAT GGCTCGAGAATACA GAGAGAAAATTGAG ACGG |
| 33 | Hs.75117 | NM_004515 | ILF2 Interleukin enhancer binding factor 2, 45kD | Homo sapiens interleukin enhancer binding factor 2, 45kD (ILF2), mRNA | TGACTTCTATTTGTG TGAAATGGCCTTTCC CCGGGTCAAGCCAG CACCTG |
| 34 | Hs.75236 | NM_021952 | ELAVL4 ELAV (embryonic lethal, abnormal vision, Drosophila)-like 4 (Hu antigen D) | Homo sapiens ELAV (embryonic lethal, abnormal vision, Drosophila)-like 4 (Hu antigen D) (ELAVL4), mRNA | GCACCATGGAGCCT CAGGTGTCAAATGG TCCGACATCCAATAC AAGCAATG |
| 35 | Hs.75258 | NM_004893 | H2AFY H2A histone family, member Y | Homo sapiens H2A histone family, member Y (H2AFY), transcript variant 2, mRNA | CACCGAAGCCAGGA AGCCCCGTTTGTAA GCGTGTGTTGTGGT GCTTTATT |
| 36 | Hs.75498 | NM_004591 | (SCYA20 Small inducible cytokine subfamily A (Cys-Cys), member 20 | Homo sapiens small inducible cytokine subfamily A (Cys-Cys), member 20 (SCYA20), mRNA | GCTACTCCACCTCT GCGGCGAATCAGAA GCAGCAAGCAACTT TGACTGCT |
| 37 | Hs.76913 | NM_002790 | PSMA5 Proteasome (prosome, macropain) subunit, alpha type, 5 | Homo sapiens proteasome (prosome, macropain) subunit, alpha type, 5 (PSMA5), mRNA | GTTTCTTACCCGGTC TGAGTACGACAGGG GCGTGAATACTTTTT CTCCCG |
| 38 | Hs.79889 | NM_012329 | MMD Monocyte to macrophage differentiation-ass ociated | Homo sapiens monocyte to macrophage differentiation-associat ed (MMD), mRNA | GCTATGAACATGCT GCTAACTGTTACACA CACGCATTCCTCATT GTTCCGGCC |
| 39 | Hs.82173 | NM_005655 | TIEG TGFB inducible early growth response | Homo sapiens TGFB inducible early growth response (TIEG), mRNA | TTTGTGGTACCCCA GCCCGTTGTGCAGA GTTCAAAGCCTCCG GTG |
| 40 | Hs. 84072 | NM_004616 | TM4SF3 Transmembrane 4 superfamily member 3 | Homo sapiens transmembrane 4 superfamily member 3 (TM4SF3), mRNA | GCAATGACTCTCAA GCAATTTTTGGTTCT GAAGATGTAGGCTC TAGCTCCTACGTTGC TGTG |
| 41 | Hs.85146 | NM_005239 | ETS2 V-ets erythroblastosis virus E26 oncogene homolog 2 (avian) | Homo sapiens v-ets erythroblastosis virus E26 oncogene homolog 2 (avian) (ETS2), mRNA | CTCATGACTCCGCC AACTGTGAATTGCCT TTGTTAACCCCGTGC AGCAAG |
| 42 | Hs.85844 | NM_002529 | NTRK1 Neurotrophic tyrosine kinase, receptor, type 1 | Homo sapiens neurotrophic tyrosine kinase, receptor, type (NTRK1), mRNA | TTCATGGACAACCCT TTCGAGTTCAACCCC GAGGACCCCATCCC TGTCT |
| 43 | Hs.88219 | NM_003454 | ZNF200 Zinc finger protein 200 | Homo sapiens zinc finger protein 200 (ZNF200), mRNA | CCCAGTCAGAAAGT CAAGGAGACCTTGG TTATTATGAAAGATG TGAGCTCAAGCCTT CAGAACAG |
| 44 | Hs.9914 | NM_006350 | FST Follistatin | Homo sapiens follistatin (FST), transcript variant FST317, mRNA | CCCTGACAGTAAGT CGGATGAGCCTGTC TGTGCCAGTGACAA TGCCACTT |
| 45 | Hs.169319 | NM_003419 | ZNF345 Zinc finger protein 345 | Homo sapiens zinc finger protein 345 (ZNF345), mRNA | CAGGGATCTCAGGA AGGACATTTCAGTGA AATGATATTTACTCC TGAAGACATGCCCA CTTTCAG |
| 46 | Hs.72805 | NM_030921 | DC42 Hypothetical protein DC42 | Homo sapiens hypothetical protein DC42 (DC42), mRNA | GGCATGGCAGCAAA TGCCAACATTTTGTG GAATAGCAGCAAAT CTACAAGAGACCCT GG |
| 47 | Hs.108301 | NM_003297 | NR2C1 Nuclear receptor subfamily 2, group C, member 1 | Homo sapiens nuclear receptor subfamily 2, group C, member 1 (NR2C1), mRNA | GACACCTACAGGTT ATCCAGACTACTACT CAGATTGCCAGCTTT AAGACTGATGAATG CTACCATC |
| 48 | Hs.177926 | NM_030941 | LOC81691 Exonuclease NEF-sp | Homo sapiens exonuclease NEF-sp (LOC81691), mRNA | CCCAGTGACGACCA AACTCAAAGATGTAC AGAGGCAGTTAAAA GCACTGCTTCCTC |
| 49 | Hs.194746 | NM_018896 | CACNA1G Calcium channel, voltage-dependen t, alpha 1G subunit | Homo sapiens calcium channel, voltage-dependent, alpha 1G subunit (CACNA1G), mRNA | ACGTCAGAGATTGT GTCTGAACCGTCCT GCTCTCTAGCTCTGA CGGATGA |
| 50 | Hs.209061 | NM_003831 | SUDD SudD suppressor of bimD6 homolog (A. nidulans) | Homo sapiens sudD suppressor of bimD6 homolog (A. nidulans) (SUDD), mRNA | TCACGGCCTGGAGT TCTTGTTCCGGACT GCAGGAATGTCTCG CAGTT |
| 51 | Hs.25087 | NM_006070 | TFG TRK-fused gene | Homo sapiens TRK-fused gene (TFG), mRNA | TAATCCTTATGCGCG TAACCGTCCTCCCTT TGGTCAGGGCTATA CCCAAC |
| 52 | Hs.3017 | NM_003284 | TNP1 Transition protein 1 (during histone to protamine replacement) | Homo sapiens transition protein 1 (duping histone to protamine replacement) (TNP1), mRNA | GATCAAAGCCAGAG AGGAGCCTATGGAA TGTGGATCAAATGC CAGTTGTGACG |
| 53 | Hs.283664 | NM_032466 | ASPH Aspartate beta-hydroxylase | Homo sapiens aspartate beta-hydroxylase (ASPH), transcript variant 3, mRNA | GAACCACAACAAGA GGATGATGAGTTTCT TATGGCGACTGATG TAGATGATAGATTTG AGACCCTGG |
| 54 | Hs.283664 | NM_032467 | ASPH Aspartate beta-hydroxylase | Homo sapiens aspartate beta-hydroxylase (ASPH), transcript variant 4, mRNA | CTCAGGGAGATGGA TTTGCTCGTTGTTTT CTTCCCTCCTTCCCC TTCCTG |
| 55 | Hs.171992 | NM_002843 | PTPRJ Protein tyrosine phosphatase, receptor type, J | Homo sapiens protein tyrosine phosphatase, receptor type, J (PTPRJ), mRNA | CCGTGGATGTGTAT GGGATTGTGTATGA CCTTCGAATGCATAG GCCTTTAATGGTGC |
| 56 | Hs.155172 | NM_003664 | AP3B1 | adaptor-related protein complex 3, beta 1 subunit | GCCCAGCTTATCATA AACACTGAGAAAACT GTGATTGGCTCTGTT CTGCTGCGGG |
| 57 | Hs.183418 | M37712 | CDC2L2 | cell dividion cycle2-like2 | CGAGAAAATGAAAA CCACCTCTTGGTTGT TCCAGAGTCACGGT TCGACCGAG |
| 58 | Hs.244473 | NM_031900 | AGXT2 | alanine-glyoxylate aminotransferase 2 | TCCGGGATTGTTACT GTCAGTGTTGGCCA TTGCCACCCAAAGG TGAATGC |
| 59 | Hs.12835 | NM_004842 | AKAP7 | A kinase (PRKA) anchor protein 7 | GAGCCCGATGACGC TGAACTAGTAAGGCT CAGTAAGAGGCTGG TGGAGAA |
| 60 | Hs.1650 | NM_000111 | SLC26A3 | solute carrier family 26, member 3 | TCAGCCCCCTATTAC ACCTGACGTGGAGA CTTTCCAAAACACCG TAGGAG |
| 61 | Hs.2998 | NM_000112 | SLC26A2 | solute carrier family 26 (sulfate transporter), member 2 | CAGCAGGGATCCAC ACACTGAAAGAAGTT CGCAGAGATTATGA AGCCATTGGAATCC |
| 62 | Hs.2246 | NM_001308 | CPN1 | carboxypeptidase N, polypeptide 1, 50kD | TCAAGTAAGCCCTGT GAGGAGAGCTCCCA GCAGAAGGCACGGA GT |
| 63 | Hs.267871 | NM_005177 | ATP6V0A1 | ATPase, H+ transporting, lysosomal V0 subunit a isoform 1 | AAATGCTTGATTGCA GAGGTCTGGTGCCC TGTCACCGACCTTG ACTCCAT |
| 64 | Hs.75445 | NM_004684 | SPARCL1 | SPARC-like 1 (mast9, hevin) | CTGCGAGCATCTCT GGTGCCCATGGAAC ACTGCATAACCCGTT TCTTTGA |
| 65 | Hs.39957 | NM_016445 | PLEK2 | pleckstrin 2 (mouse) homolog | TGGCGTTCCCACTG GGGTTAAAGGGAAT GTCCAGGGAAACCT CTTCAAAG |
| 66 | Hs.65029 | NM_002048 | GAS1 | growth arrest-specific 1 | CGACTACTACGATG AGGACTACGATGAC GAGCAGCGCACCGG |
| 67 | Hs.239926 | NM_006745 | SC4MOL | sterol-C4-methyl oxidase-like | GCTGGTTCTCGGCA TCATGATTTCCACCA CATGAACTTCATTGG AAACTATGCTTCAAC |
| 68 | Hs.59271 | NM-006758 | U2AF1 | U2(RNU2) small nuclear RNA auxiliary factor 1 | TCTGTGACAACCTG GGAGACCACCTGGT GGGGAACGTGTACG TCAAGTTT |
| 69 | Hs.8867 | NM_001554 | CYR61 | cysteine-rich, angiogenic inducer, 61 | CAAAACGCAGCCCT GCGACCACACCAAG GGGCTGGAATGCAA CTT |
| 70 | Hs.50123 | NM_003452 | ZNF189 | zinc finger protein 189 | CAACAGCGCAGTCT TGTCAACCATCAGAT GATCCATGCAGAGG TGAAAACCC |
| 71 | Hs.82071 | NM_006079 | CITED2 | Cbp/p300-interacting transactivator, with Glu/Asp-rich carboxy-terminal domain, 2 | CACCAGATGAACGG GACAAACCAGCACT TCCGAGATTGCAAC CCCAAGCA |

From the above table, the HS ID of the 71 genes comprises: Hs.107213, Hs.123107, Hs.1369, Hs.151254, Hs.1526, Hs.184270, Hs.2043, Hs.267871, Hs.4935, Hs.5509, Hs.5662, Hs.75990, Hs.83384, Hs.10029, Hs.103982, Hs.12314, Hs.150557, Hs.169266, Hs.1827, Hs.1869, Hs.194148, Hs.2352, Hs.246885, Hs.29665, Hs.3235, Hs.55209, Hs.585, Hs.62187, Hs.63290, Hs.699, Hs.74111, Hs.75103, Hs.75117, Hs.75236, Hs.75258, Hs.75498, Hs.76913, Hs.79889, Hs.82173, Hs.84072, Hs.85146, Hs.85844, Hs.88219, Hs.9914, Hs.169319, Hs.72805, Hs.108301, Hs.177926, Hs.194746, Hs.209061, Hs.25087, Hs.3017, Hs.283664, Hs.283664, Hs.171992, Hs.155172, Hs.183418, Hs.244473, Hs.12835, Hs.1650, Hs.29981, Hs.2246, Hs.267871, Hs.75445, Hs.39957, Hs.65029, Hs.239926, Hs.59271, Hs.8867, Hs.50123, Hs.82071, etc.

We obtain said specific oligonucleotides sequences by using analysis of OMP (Oligonucleotide Modeling Platform, DNA Software, Inc., Ann Arbor, MI) DNA software, Said gene sequences can act as a reagent, a biochip and a medicine for detecting colorectal cancer shown in table 1.

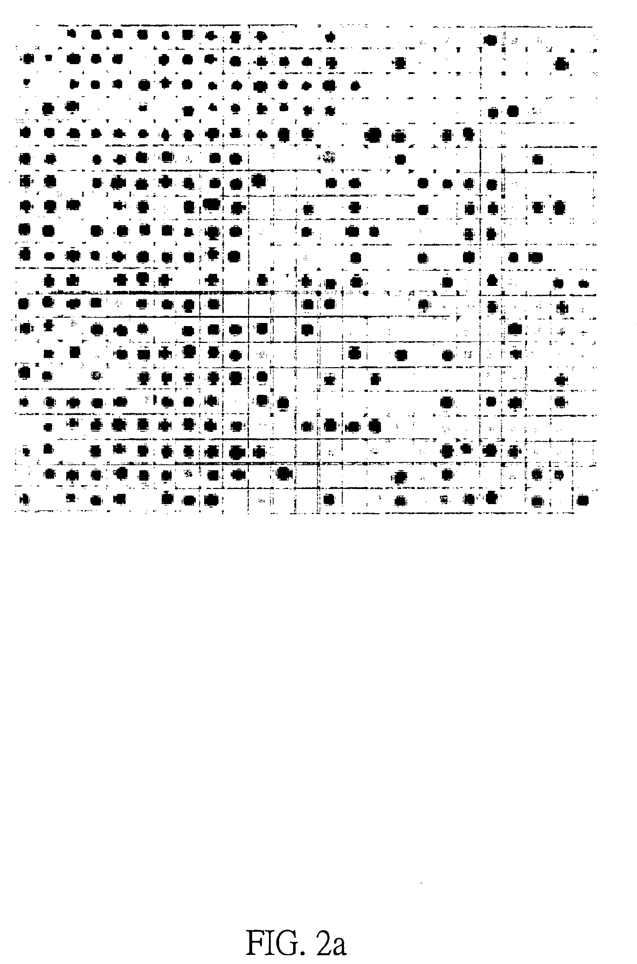

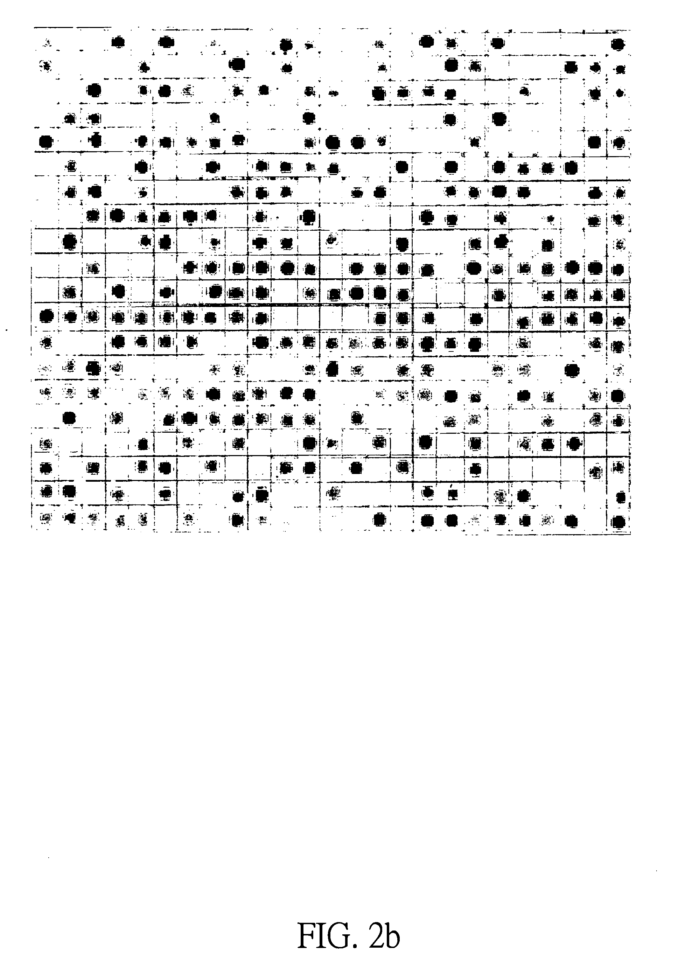

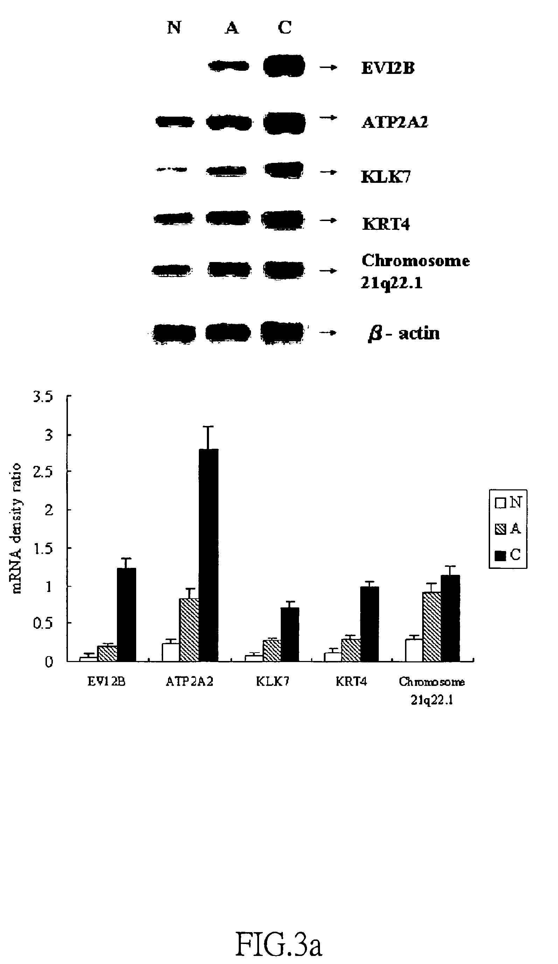

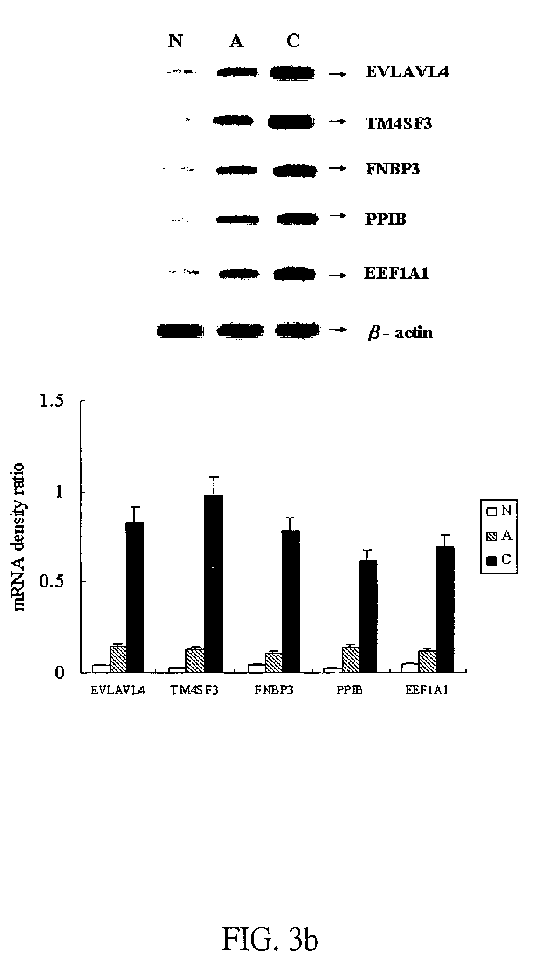

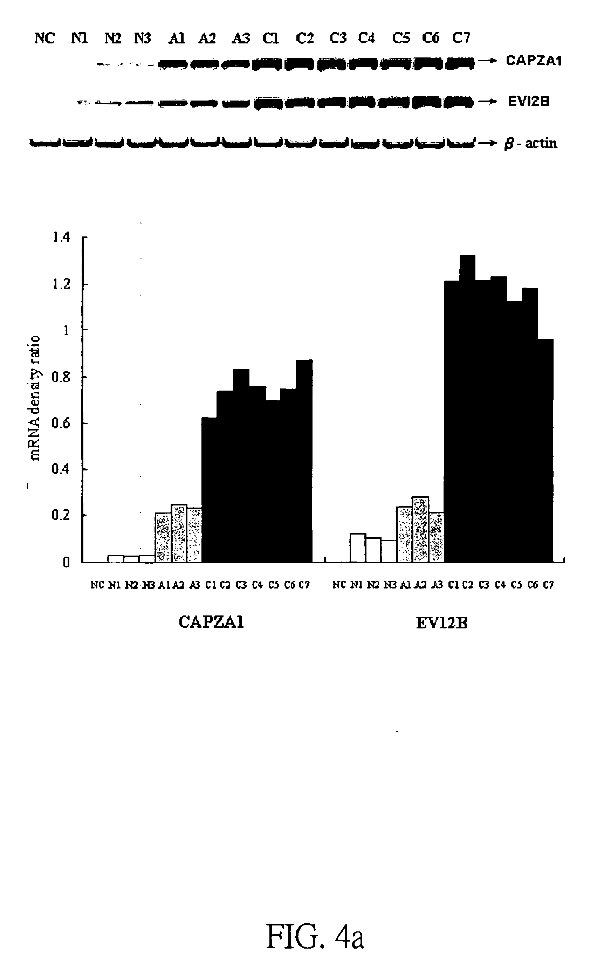

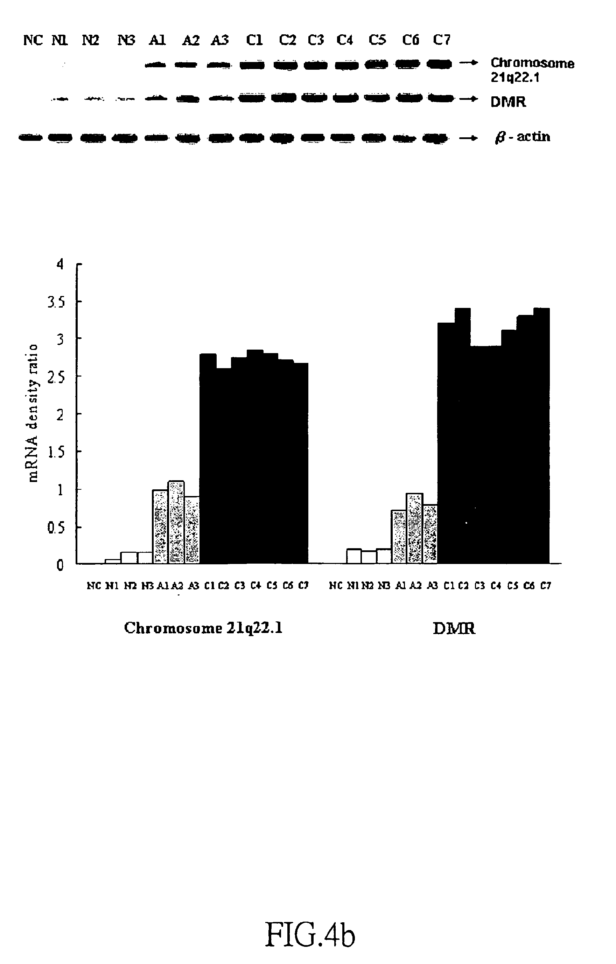

According to the present disclosure, FIG. 2a and FIG. 2b are views showing the primary screening. FIG.3a and FIG.3b are views showing affirmation to genes using Northern Blotting method. FIG.4a and 4b are views showing quantity expression of cancer tissue. we search over progressive distinctive new genes among the carcinoma process of colorectal cancer by using SSH method to build up CRA libraries and CRC libraries which make the comparison between adenoma, adenocarcinoma and normal tissue, that obtain over 5000 clones in per library; then randomly select about 3000 clones of cDNA from per library to dot on nylon membrane as pre-screen by using Colony Hybridization shown in FIG.2a and FIG.2b. The high expression colonies in colorectal cancer and adenoma are selected by the Colony Hybridization and then the nucleic acid of cDNA after purification spot on glass chip by using microarray testing.

The expression profiles of the cDNA chips were derived from a set of cDNA probes including adenoma, adenocarcinoma and the corresponding normal tissue from the same patient. Genes exhibiting at least three-fold greater intensities in the adenocarcinoma or adenoma than in corresponding normal tissue samples were considered significant. The significant up-regulated genes were then further confirmed by Northern blot (FIG. 3a and FIG. 3b) and subsequently sequenced. Northern analysis of each set of cDNA genes on the chip revealed that 36 genes were detected as up-regulated in adenoma compared to normal, and 54 genes were detected as up-regulated in carcinoma as compared to the normal control. A set of 23 genes with serial increase of genes expression from adenoma to carcinoma was identified.

Further, comparison is made by using EMBUGenBank libraries of NCBI/BLAST program, there are 3 unknown functional genes among 23 identified genes including ectopic viral integration site 2B (Genbank accession no.NM-006495) Homo sapiens chromosome 21q22.1 anonymous mRNA sequence (Genebank accession no.AF003738) and Homo sapiens DMR protein mRNA (Genbank accession no.AF327354), and another 20 functional genes. Among these 20 functional genes, 6 genes are CRC-related (such as TM4SF3), 14 genes are CRC-unrelated (such as ATP2A2). Moreover, we obtain cDNAs of three patients who suffer from adenoma and adenocarcinema simultaneously and four colorectal cancer patients to affirm variation of 23 identified genes, result shown that were at least 3-fold higher in mRNA expression level in the adenocarcinoma tissues compared with normal samples, and the level gradually increased from colorectal adenomas to adenocarcinomas shown in FIG. 4a and FIG. 4b.

Now, methods of clinical diagnosis for detecting colorectal cancer are fecal occult blood test, image test, tumor label and colonoscopy. In each of these methods, we can generalize purpose of the present disclosure according to disadvantage of these methods.

### 1. Early diagnosis

If patient undergo colorectal cancer before tumor cells spread out, five-year survival rate can be achieved over 90%. A certain number of tumor cells are needed for traditional detection by using tumor label method. In the case of image test, normally, correctly affirmation can be made easier when tumor become large. It is high invasion and price to make low acceptance for the patient in the colonoscopy that can not suitable for early diagnosis. Because of the process of circulating of tumor cells, different expression certainly happen among the genes. In the process of proliferation of early tumor cells, the dying cells cause molecule of ribonucleic acid to release into blood circulation. And, early diagnosis can be offered by the detection of using the constructed oligonucleotide biochip which is discharged from small number of tumor cells in the peripheral blood.

### Specificity and sensitivity

Fecal occult blood test has shortcomings for high false positives and false negatives to low specificity and sensitivity of the method, therefore the method is merely a first screening tool and the tumor label method is also not high specificity and sensitivity. But, we use these genes to detect peripheral blood of 100 CRC patients, peripheral blood of 50 healthy people and 40 other cancer-related patients as controls shown in FIG.1, these genes can detect 88 colorectal cancer patients for remarkable sensitivity of 88% (88/100) and specificity of 90% (90/100) in the clinical analysis.

### 3. Safety

The colonoscopy has high invasion and price to make low acceptance for patient in the mass screening tool of early diagnosis. Because sample collection is convenience and low invasion, Peripheral blood test of patient is a diagnosis method of genes, that is suitable to mass screening clinical application.

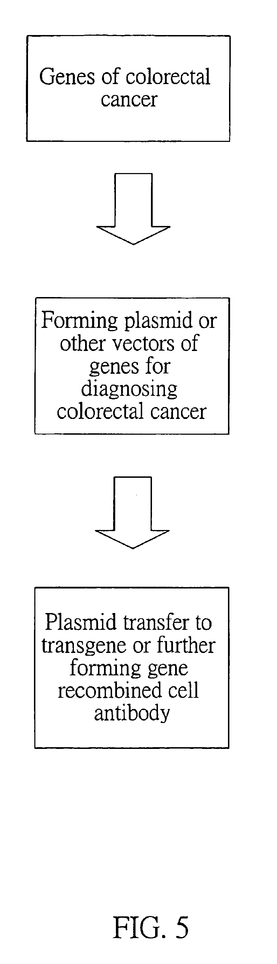

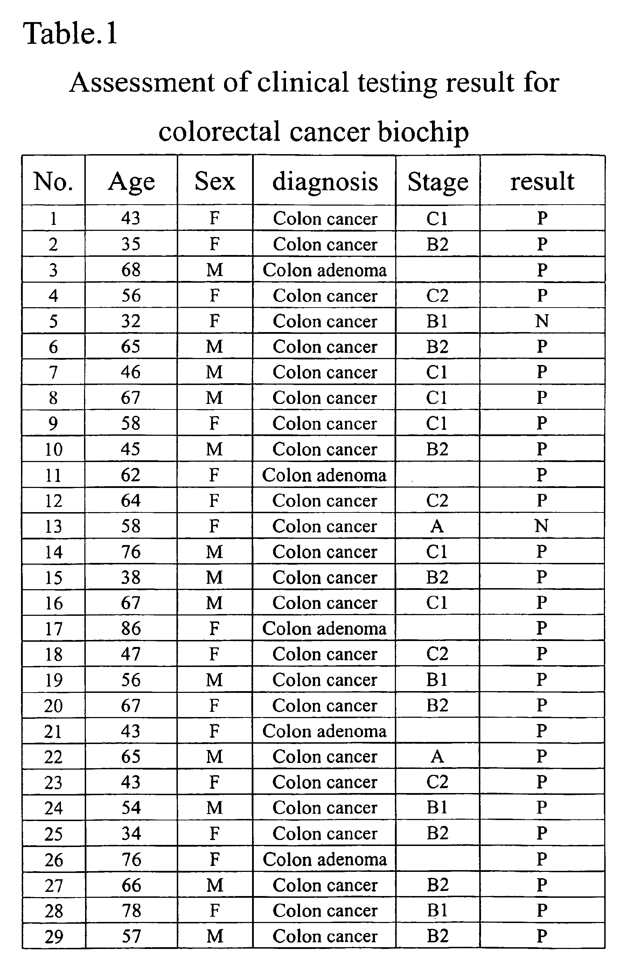

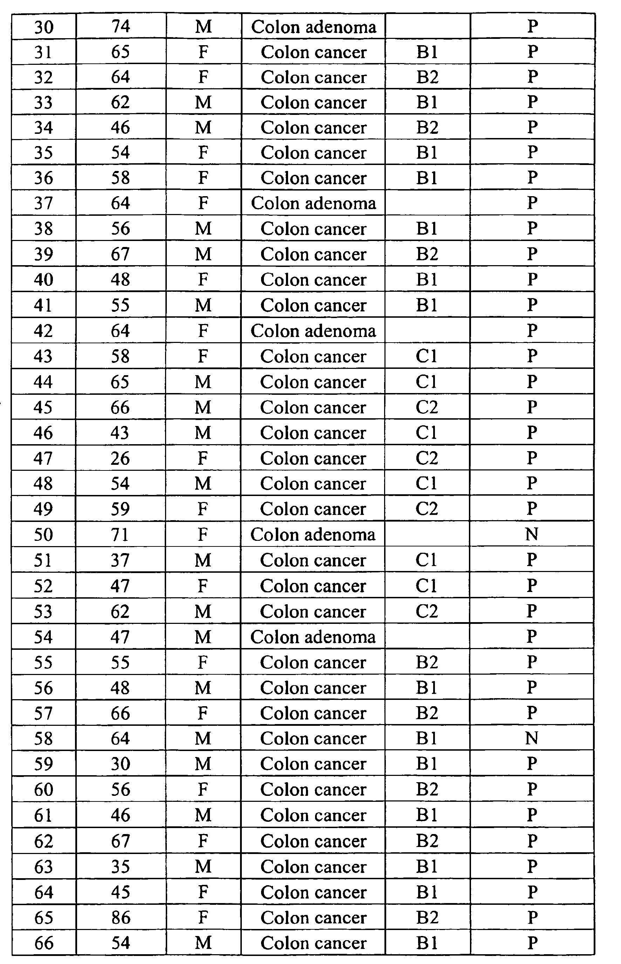

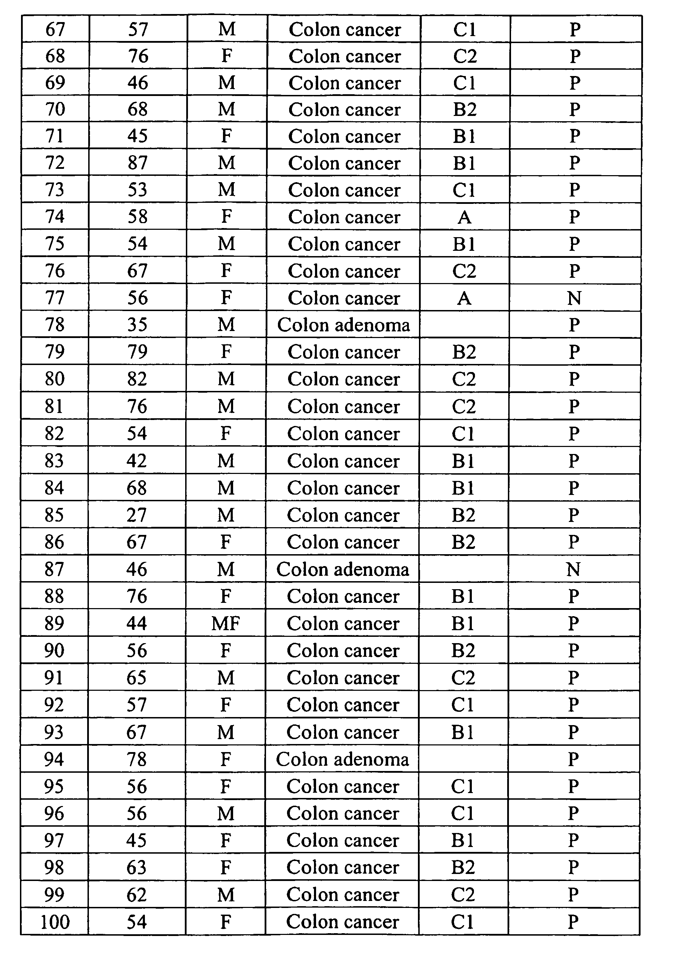

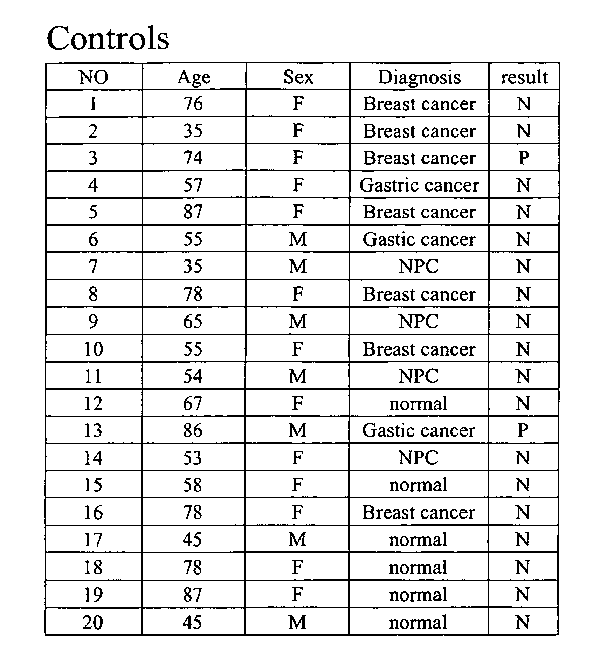

Please refer to FIG.5, showing another preferred embodiment according to the present disclosure. We choose genes of colorectal cancer and vector that express simultaneously in eukaryotic and prokaryotic to form recombination genes, and then form eukaryotic transformant cell by using and further form prokaryotic transfectant cell, and then obtain secreted protein by using extract of genes having said recombination genes, and obtain antibody from said secreted protein immune animals for making of protein testing reagent, colorectal vaccine and colorectal protein medicine for colorectal cancer.

## Claims

1. A set of oligonucleotides for diagnosing colorectal cancer consisting of oligonucleotides specific for each of the genes set forth in:
(01) a gene sequence as set forth in SEQ ID No. 1;
(02) a gene sequence as set forth in SEQ ID No. 2;
(03) a gene sequence as set forth in SEQ ID No. 3;
(04) a gene sequence as set forth in SEQ ID No. 4;
(05) a gene sequence as set forth in SEQ ID No. 5;
(06) a gene sequence as set forth in SEQ ID No. 6;
(07) a gene sequence as set forth in SEQ ID No. 7;
(08) a gene sequence as set forth in SEQ ID No. 8;
(09) a gene sequence as set forth in SEQ ID No. 9;
(10) a gene sequence as set forth in SEQ ID No. 10;
(11) a gene sequence as set forth in SEQ ID No. 11;
(12) a gene sequence as set forth in SEQ ID No. 12;
(13) a gene sequence as set forth in SEQ ID No. 13;
(14) a gene sequence as set forth in SEQ ID No. 14;
(15) a gene sequence as set forth in SEQ ID No. 15;
(16) a gene sequence as set forth in SEQ ID No. 16;
(17) a gene sequence as set forth in SEQ ID No. 17;
(18) a gene sequence as set forth in SEQ ID No. 18;
(19) a gene sequence as set forth in SEQ ID No. 19;
(20) a gene sequence as set forth in SEQ ID No. 20;
(21) a gene sequence as set forth in SEQ ID No. 21;
(22) a gene sequence as set forth in SEQ ID No. 22;
(23) a gene sequence as set forth in SEQ ID No. 23;
(24) a gene sequence as set forth in SEQ ID No. 24;
(25) a gene sequence as set forth in SEQ ID No. 25;
(26) a gene sequence as set forth in SEQ ID No. 26;
(27) a gene sequence as set forth in SEQ ID No. 27;
(28) a gene sequence as set forth in SEQ ID No. 28;
(29) a gene sequence as set forth in SEQ ID No. 29;
(30) a gene sequence as set forth in SEQ ID No. 30;
(31) a gene sequence as set forth in SEQ ID No. 31;
(32) a gene sequence as set forth in SEQ ID No. 32;
(33) a gene sequence as set forth in SEQ ID No. 33;
(34) a gene sequence as set forth in SEQ ID No. 34;
(35) a gene sequence as set forth in SEQ ID No. 35;
(36) a gene sequence as set forth in SEQ ID No. 36;
(37) a gene sequence as set forth in SEQ ID No. 37;
(38) a gene sequence as set forth in SEQ ID No. 38;
(39) a gene sequence as set forth in SEQ ID No. 39;
(40) a gene sequence as set forth in SEQ ID No. 40;
(41) a gene sequence as set forth in SEQ ID No. 41;
(42) a gene sequence as set forth in SEQ ID No. 42;
(43) a gene sequence as set forth in SEQ ID No. 43;
(44) a gene sequence as set forth in SEQ ID No. 44;
(45) a gene sequence as set forth in SEQ ID No. 45;
(46) a gene sequence as set forth in SEQ ID No. 46;
(47) a gene sequence as set forth in SEQ ID No. 47;
(48) a gene sequence as set forth in SEQ ID No. 48;
(49) a gene sequence as set forth in SEQ ID No. 49;
(50) a gene sequence as set forth in SEQ ID No. 50;
(51) a gene sequence as set forth in SEQ ID No. 51;
(52) a gene sequence as set forth in SEQ ID No. 52;
(53) a gene sequence as set forth in SEQ ID No. 53;
(54) a gene sequence as set forth in SEQ ID No. 54;
(55) a gene sequence as set forth in SEQ ID No. 55;
(56) a gene sequence as set forth in SEQ ID No. 56;
(57) a gene sequence as set forth in SEQ ID No. 57;
(58) a gene sequence as set forth in SEQ ID No. 58;
(59) a gene sequence as set forth in SEQ ID No. 59;
(60) a gene sequence as set forth in SEQ ID No. 60;
(61) a gene sequence as set forth in SEQ ID No. 61;
(62) a gene sequence as set forth in SEQ ID No. 62;
(63) a gene sequence as set forth in SEQ ID No. 63;
(64) a gene sequence as set forth in SEQ ID No. 64;
(65) a gene sequence as set forth in SEQ ID No. 65;
(66) a gene sequence as set forth in SEQ ID No. 66;
(67) a gene sequence as set forth in SEQ ID No. 67;
(68) a gene sequence as set forth in SEQ ID No. 68;
(69) a gene sequence as set forth in SEQ ID No. 69;
(70) a gene sequence as set forth in SEQ ID No. 70; and
(71) a gene sequence as set forth in SEQ ID No. 71.

2. A testing biochip for detecting colorectal cancer consisting of a set of oligonucleotides specific for each of the genes set forth in SEQ ID NOs : 1 to 71 according to claim 1.

## Patentansprüche

1. Ein Satz von Oligonukelotiden zum Diagnostizieren von Kolorektalkrebs bestehend aus Oligonukelotiden spezifisch für jedes der Gene, wie angegeben in:
(01) einer Gensequenz gemäß SEQ ID Nr.1;
(02) einer Gensequenz gemäß SEQ ID Nr.2;
(03) einer Gensequenz gemäß SEQ ID Nr.3;
(04) einer Gensequenz gemäß SEQ ID Nr.4;
(05) einer Gensequenz gemäß SEQ ID Nr.5;
(06) einer Gensequenz gemäß SEQ ID Nr.6;
(07) einer Gensequenz gemäß SEQ ID Nr.7;
(08) einer Gensequenz gemäß SEQ ID Nr.8;
(09) einer Gensequenz gemäß SEQ ID Nr.9;
(10) einer Gensequenz gemäß SEQ ID Nr.10;
(11) einer Gensequenz gemäß SEQ ID Nr.11;
(12) einer Gensequenz gemäß SEQ ID Nr.12;
(13) einer Gensequenz gemäß SEQ ID Nr.13;
(14) einer Gensequenz gemäß SEQ ID Nr.14;
(15) einer Gensequenz gemäß SEQ ID Nr.15;
(16) einer Gensequenz gemäß SEQ ID Nr.16;
(17) einer Gensequenz gemäß SEQ ID Nr.17;
(18) einer Gensequenz gemäß SEQ ID Nr.18;
(19) einer Gensequenz gemäß SEQ ID Nr.19;
(20) einer Gensequenz gemäß SEQ ID Nr.20;
(21) einer Gensequenz gemäß SEQ ID Nr.21;
(22) einer Gensequenz gemäß SEQ ID Nr.22;
(23) einer Gensequenz gemäß SEQ ID Nr.23;
(24) einer Gensequenz gemäß SEQ ID Nr.24;
(25) einer Gensequenz gemäß SEQ ID Nr.25;
(26) einer Gensequenz gemäß SEQ ID Nr.26;
(27) einer Gensequenz gemäß SEQ ID Nr.27;
(28) einer Gensequenz gemäß SEQ ID Nr.28;
(29) einer Gensequenz gemäß SEQ ID Nr.29;
(30) einer Gensequenz gemäß SEQ ID Nr.30;
(31) einer Gensequenz gemäß SEQ ID Nr.31;
(32) einer Gensequenz gemäß SEQ ID Nr.32;
(33) einer Gensequenz gemäß SEQ ID Nr.33;
(34) einer Gensequenz gemäß SEQ ID Nr.34;
(35) einer Gensequenz gemäß SEQ ID Nr.35;
(36) einer Gensequenz gemäß SEQ ID Nr.36;
(37) einer Gensequenz gemäß SEQ ID Nr.37;
(38) einer Gensequenz gemäß SEQ ID Nr.38;
(39) einer Gensequenz gemäß SEQ ID Nr.39;
(40) einer Gensequenz gemäß SEQ ID Nr.40;
(41) einer Gensequenz gemäß SEQ ID Nr.41;
(42) einer Gensequenz gemäß SEQ ID Nr.42;
(43) einer Gensequenz gemäß SEQ ID Nr.43;
(44) einer Gensequenz gemäß SEQ ID Nr.44;
(45) einer Gensequenz gemäß SEQ ID Nr.45;
(46) einer Gensequenz gemäß SEQ ID Nr.46;
(47) einer Gensequenz gemäß SEQ ID Nr.47;
(48) einer Gensequenz gemäß SEQ ID Nr.48;
(49) einer Gensequenz gemäß SEQ ID Nr.49;
(50) einer Gensequenz gemäß SEQ ID Nr.50;
(51) einer Gensequenz gemäß SEQ ID Nr.51;
(52) einer Gensequenz gemäß SEQ ID Nr.52;
(53) einer Gensequenz gemäß SEQ ID Nr.53;
(54) einer Gensequenz gemäß SEQ ID Nr.54;
(55) einer Gensequenz gemäß SEQ ID Nr.55;
(56) einer Gensequenz gemäß SEQ ID Nr.56;
(57) einer Gensequenz gemäß SEQ ID Nr.57;
(58) einer Gensequenz gemäß SEQ ID Nr.58;
(59) einer Gensequenz gemäß SEQ ID Nr.59;
(60) einer Gensequenz gemäß SEQ ID Nr.60;
(61) einer Gensequenz gemäß SEQ ID Nr.61;
(62) einer Gensequenz gemäß SEQ ID Nr.62;
(63) einer Gensequenz gemäß SEQ ID Nr.63;
(64) einer Gensequenz gemäß SEQ ID Nr.64;
(65) einer Gensequenz gemäß SEQ ID Nr.65;
(66) einer Gensequenz gemäß SEQ ID Nr.66;
(67) einer Gensequenz gemäß SEQ ID Nr.67;
(68) einer Gensequenz gemäß SEQ ID Nr.68;
(69) einer Gensequenz gemäß SEQ ID Nr.69;
(70) einer Gensequenz gemäß SEQ ID Nr.70; und
(71) einer Gensequenz gemäß SEQ ID Nr.71.

2. Ein Test-Biochip zum Erkennen von Kolorektalkrebs bestehend aus einem Satz von Oligonukelotiden spezifisch für jedes der Gene wie angegeben in SEQ ID Nr.1 bis 71 gemäß Anspruch 1.

## Revendications

1. Un ensemble d'oligonucléotides pour diagnostiquer le cancer côlorectal se composant des oligonucléotides spécifiques pour chacun des gènes a déterminé dans
(01) une séquence de gène comme déterminé dans SEQ ID No. 1;
(02) une séquence de gène comme déterminé in SEQ ID No. 2;
(03) une séquence de gène comme déterminé in SEQ ID No. 3;
(04) une séquence de gène comme déterminé in SEQ ID No. 4;
(05) une séquence de gène comme déterminé in SEQ ID No. 5;
(06) une séquence de gène comme déterminé in SEQ ID No. 6;
(07) une séquence de gène comme déterminé in SEQ ID No. 7;
(08) une séquence de gène comme déterminé in SEQ ID No. 8;
(09) une séquence de gène comme déterminé in SEQ ID No. 9;
(10) une séquence de gène comme déterminé in SEQ ID No. 10;
(11) une séquence de gène comme déterminé in SEQ ID No. 11;
(12) une séquence de gène comme déterminé in SEQ ID No. 12;
(13) une séquence de gène comme déterminé in SEQ ID No. 13;
(14) une séquence de gène comme déterminé in SEQ ID No. 14;
(15) une séquence de gène comme déterminé in SEQ ID No. 15;
(16) une séquence de gène comme déterminé in SEQ ID No. 16;
(17) une séquence de gène comme déterminé in SEQ ID No. 17;
(18) une séquence de gène comme déterminé in SEQ ID No. 18;
(19) une séquence de gène comme déterminé in SEQ ID No. 19;
(20) une séquence de gène comme déterminé in SEQ ID No. 20;
(21) une séquence de gène comme déterminé in SEQ ID No. 21;
(22) une séquence de gène comme déterminé in SEQ ID No. 22;
(23) une séquence de gène comme déterminé in SEQ ID No. 23;
(24) une séquence de gène comme déterminé in SEQ ID No. 24;
(25) une séquence de gène comme déterminé in SEQ ID No. 25;
(26) une séquence de gène comme déterminé in SEQ ID No. 26;
(27) une séquence de gène comme déterminé in SEQ ID No. 27;
(28) une séquence de gène comme déterminé in SEQ ID No. 28;
(29) une séquence de gène comme déterminé in SEQ ID No. 29;
(30) une séquence de gène comme déterminé in SEQ ID No. 30;
(31) une séquence de gène comme déterminé in SEQ ID No. 31;
(32) une séquence de gène comme déterminé in SEQ ID No. 32;
(33) une séquence de gène comme déterminé in SEQ ID No. 33;
(34) une séquence de gène comme déterminé in SEQ ID No. 34;
(35) une séquence de gène comme déterminé in SEQ ID No. 35;
(36) une séquence de gène comme déterminé in SEQ ID No. 36;
(37) une séquence de gène comme déterminé in SEQ ID No. 37;
(38) une séquence de gène comme déterminé in SEQ ID No. 38;
(39) une séquence de gène comme déterminé in SEQ ID No. 39;
(40) une séquence de gène comme déterminé in SEQ ID No. 40;
(41) une séquence de gène comme déterminé in SEQ ID No. 41;
(42) une séquence de gène comme déterminé in SEQ ID No. 42;
(43) une séquence de gène comme déterminé in SEQ ID No. 43;
(44) une séquence de gène comme déterminé in SEQ ID No. 44;
(45) une séquence de gène comme déterminé in SEQ ID No. 45;
(46) une séquence de gène comme déterminé in SEQ ID No. 46;
(47) une séquence de gène comme déterminé in SEQ ID No. 47;
(48) une séquence de gène comme déterminé in SEQ ID No. 48;
(49) une séquence de gène comme déterminé in SEQ ID No. 49;
(50) une séquence de gène comme déterminé in SEQ ID No. 50;
(51) une séquence de gène comme déterminé in SEQ ID No. 51;
(52) une séquence de gène comme déterminé in SEQ ID No. 52;
(53) une séquence de gène comme déterminé in SEQ ID No. 53;
(54) une séquence de gène comme déterminé in SEQ ID No. 54;
(55) une séquence de gène comme déterminé in SEQ ID No. 55;
(56) une séquence de gène comme déterminé in SEQ ID No. 56;
(57) une séquence de gène comme déterminé in SEQ ID No. 57;
(58) une séquence de gène comme déterminé in SEQ ID No. 58;
(59) une séquence de gène comme déterminé in SEQ ID No. 59;
(60) une séquence de gène comme déterminé in SEQ ID No. 60;
(61) une séquence de gène comme déterminé in SEQ ID No. 61;
(62) une séquence de gène comme déterminé in SEQ ID No. 62;
(63) une séquence de gène comme déterminé in SEQ ID No. 63;
(64) une séquence de gène comme déterminé in SEQ ID No. 64;
(65) une séquence de gène comme déterminé in SEQ ID No. 65;
(66) une séquence de gène comme déterminé in SEQ ID No. 66;
(67) une séquence de gène comme déterminé in SEQ ID No. 67;
(68) une séquence de gène comme déterminé in SEQ ID No. 68;
(69) une séquence de gène comme déterminé in SEQ ID No. 69;
(70) une séquence de gène comme déterminé in SEQ ID No. 70; et
(71) une séquence de gène comme déterminé in SEQ ID No. 71.

2. Une biopuce d'essai pour détecter le cancer côlorectal se composer d'un ensemble d'oligonucléotides spécifiques pour chacun des gènes a déterminé dans SEQ ID Nos. 1 à 71 selon la revendication 1.
